# NEUE EUROPÄISCHE PATENTSCHRIFT

(11) **EP 1 478 662 B2**
(45) Veröffentlichungstag und Bekanntmachung des Hinweises auf die Entscheidung über den Einspruch: **05.07.2017**
(45) Hinweis auf die Patenterteilung: 23.09.2009
(21) Anmeldenummer: 03706458.1
(22) Anmeldetag: 07.02.2003
(51) Int. Cl.: C07K 14/415, C07K 1/36, C07K 1/113

(54) **VERFAHREN ZUR HERSTELLUNG VON HYPOALLERGENEM BIRKENPOLLENHAUPTALLERGEN RBET V 1**
METHOD FOR PRODUCING HYPOALLERGENIC MAJOR BIRCH POLLEN ALLERGENS RBET V 1
PROCEDE POUR PRODUIRE DES ALLERGENES MAJEURS DES POLLENS DE BOULEAU HYPOALLERGENIQUES RBET V 1

(30) Priorität: 27.02.2002 EP 02004567
(43) Veröffentlichungstag der Anmeldung: 24.11.2004
(73) Patentinhaber: Merck Patent GmbH, 64293 Darmstadt (DE)
(72) Erfinder: SUCK, Roland, 22301 Hamburg (DE); FIEBIG, Helmut, 21493 Schwarzenbek (DE); CROMWELL, Oliver, 21465 Wentorf (DE)
(86) Internationale Anmeldenummer: PCT/EP2003/001246
(87) Internationale Veröffentlichungsnummer: WO 2003/072601

(56) Entgegenhaltungen:
- WO-A-02/20559
- WO-A1-02/20559
- HOFFMANN-SOMMERGRUBER KARIN ET AL: "High-level expression and purification of the major birch pollen allergen, Bet v 1." PROTEIN EXPRESSION AND PURIFICATION, Bd. 9, Nr. 1, 1997, Seiten 33-39, XP002240372 ISSN: 1046-5928 in der Anmeldung erwähnt
- VRTALA S ET AL: "Conversion of the major birch pollen allergen, bet v 1, into two nonanaphylactic T cell epitope-containing fragments: Candidates for a novel form of specific immunotherapy" JOURNAL OF CLINICAL INVESTIGATION, NEW YORK, NY, US, Bd. 99, Nr. 7, 1997, Seiten 1673-1681, XP002221492 ISSN: 0021-9738
- SINGH M B ET AL: "GENETICALLY ENGINEERED PLANT ALLERGENS WITH REDUCED ANAPHYLACTIC ACTIVITY" INTERNATIONAL ARCHIVES OF ALLERGY AND IMMUNOLOGY, XX, XX, Bd. 119, Nr. 2, 1999, Seiten 75-85, XP001010229 ISSN: 1018-2438 in der Anmeldung erwähnt
- HOFFMANN-SOMMERGRUBER K. ET AL: 'High-level expression and purification of the major birch pollen allergen, Bet v 1' PROTEIN EXPRESSION AND PURIFICATION Bd. 9, 1997, Seiten 33 - 39
- FRIEDL-HAJEK R. ET AL: 'New Bet v 1 isoforms including a naturally occurring truncated form of the protein derived from Austrian birch pollen' MOLECULAR IMMUNOLOGY Bd. 36, 1999, Seiten 639 - 645
- GAJHEDE M. ET AL: 'X-ray and NMR structure of Bet v 1, the origin of birch pollen allergy' NATURE STRUCTURAL BIOLOGY Bd. 3, Dezember 1996, Seiten 1040 - 1045
- SUCK R. ET AL: 'Purification and immunobiochemical characterization of folding variants of the recombinant major wasp allergen Ves v 5 (Antigen 5)' INTERNATIONAL ARCHIVES OF ALLERGY AND IMMUNOLOGY Bd. 121, 2000, Seiten 284 - 291
- European patent appliacation No: 02004567; priority document for the Opposed patent
- LEHNINGER A.L.: 'Principles of Biochemistry', 1982, WORTH PUBLISHERS Seite 140
- KAHLERT H. ET AL: 'Characterizaton of a Hypoallergenic recombinant Bet v 1 variant as a candidate for allergen-specific immunotherapy' INT. ARCH. ALLERGY IMMUNOL. Bd. 145, 2008, Seiten 193 - 206
- JANSON J-C. ET AL: 'Protein Purification', Bd. 2. EDIT., 1998, JOHN WILEY & SONS, INC. Seite 86
- WALSH G.: 'Biopharmaceuticals: Biochemistry and Biotechnology', 1998, JOHN WILEY & SONS LTD Seite 8

## Beschreibung

### Technisches Gebiet der Erfindung

Die Erfindung betrifft ein Verfahren zur Herstellung von Birkenpollenallergenen, die sich durch eine fehlende, zumindest jedoch durch eine reduzierte Immunglobulin E - Bindung, das heißt durch Hypoallergenität auszeichnen. Diese Allergene behalten die therapeutisch relevante T-Zell-Stimulierung vollständig. Deshalb können sie als nebenwirkungsarme Therapeutika zur spezifischen Immuntherapie eingesetzt werden.

### Hintergrund der Erfindung

Allergien vom Typ 1 haben in den letzten Jahrzehnten weltweit dramatisch zugenommen. Bis zu 20% der Bevölkerung in industrialisierten Ländern leiden unter Beschwerden, wie allergischer Rhinitis, Konjunktivitis oder Bronchialasthma, die durch in der Luft befindliche Allergene (Aeroallergene), die von unterschiedlichen Quellen wie Pflanzenpollen, Milben, Säugetieren (Katzen, Hunden, Pferden) und Schimmelpilzen freigesetzt werden, hervorgerufen werden. Schwerwiegende Allergien können auch durch Stiche von Insekten, wie z.B. von Bienen und Wespen, ausgelöst werden.

Bei den Typ 1 -Allergie auslösenden Substanzen handelt es sich um Proteine, Glykoproteine oder Polypeptide. Diese Allergene reagieren nach Aufnahme über die Schleimhäute oder nach Stichen mit den bei sensibilisierten Personen an der Oberfläche von Mastzellen gebundenen IgE-Antikörpern. Werden zwei oder mehr IgE-Antikörpern durch ein Allergen miteinander vernetzt, führt dies zur Ausschüttung von Mediatoren (z.B. Histamin, Prostaglandinen) und Zytokinen durch die Effektorzelle und damit zur Auslösung der allergischen Symptome.

Birkenpollen sind unter den Baumpollen die häufigsten Auslöser allergischer Reaktionen (Jarolim E. et al., 1989, Allergy 44:385-95). Mehr als 90% der unter Birkenpollenallergien leidenden Menschen besitzen IgE-Antikörper gegen das Hauptallergen Bet v 1 (Elfman, L. et al., 1997, Int. Arch. Allergy Immunol., 113: 249-51).

Mit Hilfe von cDNA-Sequenzen ist es möglich, rekombinante Allergene herzustellen, die in der Diagnostik und Therapie von Allergien Verwendung finden können. (Scheiner und Kraft, 1995, Allergy 50, 384-391). Die Herstellung rekombinanter Bet v 1 Allergene (rBet v 1) und ihre Reinigung für pharmazeutische Zwecke wurde z.B. von Hoffmann-Sommergruber et al. (Protein Exp. Purif. 9 (1), 1997: 33-39) beschrieben.

Darüber hinaus sind gezielte gentechnische Veränderungen der rekombinanten Allergene möglich, wodurch ein reduziertes allergenes Potenzial erreicht werden kann (Schramm et al., 1999, J. Immunol. 162 (4): 2406-2414; Valenta et al., 1999, Biol. Chem. 380: 815-24; Singh et al., 1999, Int. Arch. Allergy Immunol. 119: 75-85). Solche Allergenvarianten sind aussichtsreiche zukünftige Kandidaten für die spezifische Immuntherapie der Typ 1-Allergie.

Ein potentieller Nachteil bei rekombinanten Allergenvarianten ist jedoch, daß durch die Veränderung der Primärsturktur die für den therapeutischen Erfolg notwendigen T-Zell Epitope verloren gehen oder in ihrer Reaktivität abnehmen. Diese Möglichkeit kann nur ausgeschlossen werden, wenn die dem natürlichen Allergen entsprechende Primärstruktur als Basis zur Herstellung des rekombinanten Proteins dient.

Im Falle des Birkenpollenhauptallergens Bet v 1 wurde, um es für Therapiezwecke rekombinant zu optimieren, d.h. die IgE-Bindefähigkeit zu reduzieren, in zwei Hälften (Vrtala, S., et al., 1997, J. Clin. Invest. 99: 1673-81) oder als Trimer (Vrtala, S., et al., 1999, Int. Arch. Allergy Immunol. 118:218-9) hergestellt. Nachteilig wirkt sich auch bei diesen Ansätzen der potentielle Verlust von T-Zell-Epitopen sowie die Unlöslichkeit der Proteine aus. Ein weiterer Nachteil ist hier in der aufwendigen Art der Herstellung dieser rBet v 1 Varianten zu sehen.

Ein geeigneter Ansatz zur Herstellung eines rekombinanten Hauptallergens rBet v 1, das für therapeutische Zwecke genutzt werden kann, wäre demnach ein dem Wildtyp in der Primärstruktur entsprechendes, in seiner T-Zell-Stimulierung uneingeschränktes, aber vermindert IgE-aktives, das heißt hypoallergenes Molekül.

Diese Aufgabe wurde im Rahmen der vorliegenden Erfindung durch die Ausführung einer Reihe von an sich bekannten biochemischen Reinigungsschritte unter Verwendung von löslichem rekombinantem Hauptallergen rBet v 1 als Ausgangsstoff gelöst.

Überraschenderweise wurde bei den so gereinigten Proteinen eine verringerte IgE-Aktivität und zugleich erhaltene T-Zell-Stimulierung festgestellt.

Demnach liefert das erfindungsgemäße Verfahren eine verbesserte therapeutische Wirksamkeit bei gleichzeitiger deutlicher Reduzierung bzw. Abwesenheit von Nebenwirkungen.

Hierbei ist die Ausgestaltung des Herstellungsverfahrens der rekombinanten Allergene insofern von besonderer Bedeutung, als im Zuge dieses Verfahrens die Proteine in eine Konformation überführt werden, die keine oder eine stark verminderte Affinität zu IgE bei gleichbleibender T-Zell-Stimulierung aufweist.

### Abbildungen

### Abbildung 1A: SDS-PAGE zur Charakterisierung des hypoallergenen rBet v 1

Spur 1: Protein-Standard zur Abschätzung des Molekulargewichts
Spur 2: Natürliches nBet v 1
Spur 3: Erfindungsgemäß gereinigtes rBet v 1
Spur 4: Konventionell gereinigtes rBet v 1

### Abbildung 1B: Nitrozellulose-Blott der SDS-PAGE aus Abb. 1A

### Abbildung 2A: Nitrozellulose-Blot zur Bestimmung der IgE-Aktivität mit 20 individuellen Patientenseren

Position 3: Natürliches nBet v 1
Position 4: Erfindungsgemäß gereinigtes rBet v 1
Position 5: Konventionell gereinigtes rBet v 1

### Abbildung 2B: Nitrozellulose-Blot zur Bestimmung der Identität der Bet v 1-Proben

Blots 21-26: verschiedene polyklonale Kaninchen-Anti-Bet v 1-Antikörper
Blot 27: monoklonaler Maus-Anti-Bet v 1-Antikörper 6B6

### Abbildung 3: Enzym-Allergo-Sorbent-Test (EAST) zur Quantifizierung der IgE-Bindung

Auf der vertikalen Achse ist die Konzentration eines Inhibitors der IgE-Bet v 1-Bindung in µg/ml aufgetragen, auf der horizontalen Achse ist der Grad der Inhibierung in [%] angegeben.

### Abbildung 4: Bestimmung der T-Zell-Stimulierung von Bet v 1-Varianten

Gegenübergestellt sind die Konzentrationen des natürlichen nBet v 1, des herkömmlich gereinigten rekombinanten rBet v 1 sowie des erfindungsgemäß gereinigten rekombinanten rBet v 1 und die jeweiligen mit verschiedenen T-Zell-Linien (TCL) und T-Zell-Klonen (TCC) erhaltenen Stimulationsindices (SI).

### Detaillierte Beschreibung der Erfindung

Bei der vorliegenden Erfindung handelt es sich um ein biochemisches Reinigungsverfahren, das über eine effiziente Reinigung von beispielsweise rekombinant hergestellten Allergenen unter Verwendung spezieller Laufmittel zur Darstellung von Proteinen mit den erfindungsgemäß veränderten Eigenschaften führt.

Diese Eigenschaften bestehen in einer fehlenden, zumindest aber in einer stark reduzierten IgE-Aktivität, bei gleichzeitigem Erhalt der T-Zell-Stimulierung.

Gegenstand der Erfindung ist somit ein Verfahren zur Reduzierung der IgE-Aktivität des Birkenpollenhauptallergens rBet v 1, welches in der Verwendung von löslichem rekombinantem Birkenpollenhauptallergen rBet v 1 sowie in der Ausführung der nachfolgend beschriebenen Chromatographieschritte zu seiner Reinigung und des anschließenden Neutralisierungsschritts besteht.

Gegenstand der Erfindung ist ferner ein Verfahren zur Herstellung von hypoallergenem Birkenpollenhauptallergen rBet v 1 mittels mehrerer chromatographischer Reinigungsschritte unter Verwendung von im wesentlichen ungepufferten wässrigen Basen als Laufmittel und anschließender Neutralisation, wobei als Ausgangsstoff ein rekombinant hergestelltes lösliches rBet v 1 Rohprotein eingesetzt wird.

Die chromatographischen Reinigungsschritte umfassen vorzugsweise Anionenaustausch-Chromatographie, Hydrophobe Interaktions-Chromatographie sowie Gelfiltration und können einmal, mehrmals hinter einander oder mehrfach abwechselnd ausgeführt werden. Vorzugsweise werden die chromatographischen Reinigungsschritte jedoch in folgender Reihenfolge ausgeführt: erste Gelfiltration, Anionenaustausch-Chromatographie, Hydrophobe Interaktions-Chromatographie, zweite Gelfiltration.

Üblicherweise erfolgt die chromatographische Reinigung mit einer Basenkonzentration von 5 bis 100 mM, vorzugsweise jedoch mit 5 bis 40 mM und besonders bevorzugt mit 10 bis 30 mM, wobei als basische Substanz bevorzugt NaOH eingesetzt wird. Anstelle eines rein wässrigen Systems kann auch ein Mischsystem verwendet werden, das beispielsweise Wasser und Methanol enthält. Auch ein nicht-wässriges, z.B. methanolisches System ist denkbar. Bevorzugt wird jedoch mit einem wässrigem System gearbeitet.

In Abhängigkeit von dem jeweiligen Chromatographieschritt können unterschiedliche Konzentrationen eines Neutralsalzes - bevorzugt NaCl - bis etwa 5 M dem Laufmittel zugesetzt werden.

Zur gegebenenfalls erforderlichen Absenkung des pH-Wertes auf Werte, die von empfindlicheren Proteinen toleriert werden, kann Natriumhydrogencarbonat zugegeben werden.

Auch die Absicht, am Ende der Reinigung physiologische Bedingungen einzustellen, kann ein Grund für die Anwesenheit von Natriumhydrogencarbonat während der Chromatographieschritte sein. Hierbei sind grundsätzlich Konzentrationen von bis zu 100 mM möglich. Bevorzugt wird aber im physiologischen Bereich von unter 20 mM, besonders bevorzugt bei 11 mM gearbeitet.

Im Falle von rBet v 1 ist eine Absenkung des pH-Werts nicht erforderlich. Dennoch wird bei der Anwendung des erfindungsgemäßen Verfahrens auf rBet v 1 in der Regel Natriumhydrogencarbonat zugesetzt, um am Ende der Reinigung auf einfache Weise physiologische Konzentrationen einstellen zu können.

Bei der Erfindung handelt es sich somit um ein Verfahren zur Herstellung von hypoallergenem Birkenpollenhauptallergen rBet v 1, worin im wesentlichen ungepufferte basische Laufmittel die zu reinigenden Proteine schonend in Lösung und damit in einem chromatographierbaren Zustand erhalten.

In einer bevorzugten Ausführungsform des Verfahrens wird das rBet v 1 Rohprotein vor der eigentlichen Reinigung durch eine Chromatographie, beispielsweise eine Hydrophobe Interaktions-Chromatographie oder eine lonenaustausch-Chromatographie und/oder eine Salzfällung vorgereinigt, wobei im Gegensatz zur anschließenden Hauptreinigung mit einem gepufferten Laufmittel bzw. einer gepufferten Lösung gearbeitet wird.

Die Ausgangsstoffe für das Verfahren bilden in Bakterien oder anderen geeigneten Wirtszellen (wie etwa Hefen) exprimierte, lösliche rekombinante Allergene. Da das Verfahren eine für diese Expressionsprodukte generelle Applikation darstellt, können erfindungsgemäss auch lösliche Allergene anderer Herkunft nach dem Verfahren gereinigt, renaturiert und formuliert werden. Insbesondere bei einer entsprechenden Ähnlichkeit dieser Allergene anderer Herkunft mit dem Birkenpollenhauptallergen Bet v 1 ist mit der Erzielung der erfindungsgemäßen Eigenschaften zu rechnen.

Proteins oder seiner Fragmente mit anderen Proteinen oder Peptiden in Frage kommen. Die Modifikationen können aber auch chemischer Natur sein und auf Proteinebene erfolgen.

Nachfolgend wird das erfindungsgemäße HerstellungsverFahren in allgemeiner Form beschrieben. Dabei stammen sämtliche, beispielhaft erwähnten Chromatographiematerialen von der Firma Amersham Biosciences (Freiburg, Deutschland).

Ein erster Vorreinigungsschritt kann zur Entfernung von Nukleinsäuren in einer unter physiologischen Bedingungen (bei einem pH von 6-8, nicht denaturierend) durchgeführten Hydrophobe Interaktions-Chromatographie bestehen, wobei das Zielprotein gleichzeitig fokussiert wird. Alternativ dazu können auch eine Salzfällung oder eine lonenaustausch-Chromatographie durchgeführt werden. Die Durchführung dieses ersten Vorreinigungsschritts ist für die Erzielung des erfindungsgemäßen Effekts jedoch nicht zwingend erforderlich.

Der nächste Aufreinigungschritt dient zur Überführung der Proteine in schwach salines Laufmittel im Konzentrationsbereich von 10-100 mM, z.B. 20 mM NaCl, beispielsweise mittels Gelfiltration auf einer Sephadex G-25 Säule. Dadurch werden Bedingungen geschaffen, die die Durchführung einer Ionenaustausch-Chromatograhie mit alkalischen Laufmitteln ermöglichen. Die so vorbereitete Proteinlösung wird im folgenden zur Anionen-Austauschchromatographie, beispielsweise mit einer Source Q Säule, eingesetzt. Hierbei werden die meisten Allergene an den Träger gebunden. Das alkalische Laufmittel bewirkt, daß auch zuvor schwer- oder unlösliche Proteine in Lösung verbleiben. Durch NaCl-Gradientenelution erfolgt eine partielle Abtrennung von bakteriellen Verunreinigungen und Wirkstofffragmenten.

In zwei weiteren Aufreinigungsschritten, einer Hydrophobe Interaktions-Chromatographie und einer Gelfiltration, werden die vorgereinigten und äquilibrierten Allergene im wesentlichen von noch verbliebenen bakteriellen Verunreinigungen abgetrennt. Hierzu werden grundsätzlich die gleichen Laufmittelsubstanzen benutzt, bestehend aus niedermolarer Base und einem variierenden Anteil eines anorganischen Neutralsalzes. So können die Allergene bei der Hydrophobe Interaktions-Chromatographie mit z.B. bis zu 5 M NaCl, 20 mM NaOH und 11 mM NaHCO₃ an die Säule gebunden und anschließend mit salzarmer oder salzfreier alkalischer Lösung, z.B. 20 mM NaOH eluiert werden.

Im letzten Chromatographieschritt erfolgt ein Laufmittelwechsel dergestalt, daß die gereinigten rekombinanten Proteine durch einfache Neutralisation der im Laufmittel vorhandenen Base mit einer entsprechenden Säure in löslicher gebrauchsfähiger Form gewonnen werden. Bei geeigneter Wahl der Konzentrationen der Laufmittelzusätze entsteht eine für Parenteralia geeignete physiologische Lösung.

Die Identifikation der gereinigten Allergene erfolgt über ihre bekannten physikalischen, chemischen oder biologischen Eigenschaften, insbesondere mittels SDS-PAGE und spezifischer monoklonaler Antikörper. Zur weiteren Charakterisierung kann beispielsweise ein EAST-Inhibitionsassay (EAST bedeutet Enzym-Aliergo-Sorbent-Test), mit dem die spezifische IgE-Bindung eines Proteins im Vergleich zu einer Referenz bestimmt werden kann, und/oder ein T-Zell-Proliferationsassay durchgeführt werden.

Die Prüfung des Lösungsmittels erfolgt durch pH-Wert Messung und Quantifizierung der Na⁺- und Cl⁻ sowie gegebenenfalls CO₃²⁻ -Konzentration. Diese Verfahren sind allgemein bekannt und beschrieben.

Die Ausbeute der erfindungsgemäss hergestellten Allergene beträgt im Allgemeinen 75-95 % bezogen auf das Ausgangsprotein.

Das Verfahren beinhaltet somit eine minimale Probenbehandlungen, kurze Probenstandzeiten, vorzugsweise die Verwendung von ausschliesslich pharmakologisch kompatiblen Substanzen, die Kompatibilität eines einzigen Laufmittels mit diversen Trennprinzipien, und die Umgehung von langwierigen und unter Umständen nicht validierbaren Verfahren wie Dialysen. Zudem verhindert die als Base bevorzugt eingesetzte Natronlauge, die als effektives Bakteriostatikum bekannt ist, dass die in ihr vorhandenen Proteine durch Mirkroorganismen degradiert bzw. verunreinigt werden. Ebenso werden Endotoxine, die bei bakteriellen Expressionen problematisch sein können, sonstige Fremdproteine und DNA effektiv entfernt bzw. degradiert.

Die Reihenfolge und die Anzahl der oben beschriebenen Chromatographieschritte kann verändert werden. Damit kann unter anderem den spezifischen physikochemischen Eigenschaften der Zielproteine Rechnung getragen werden.

Auch ohne weitere Ausführungen wird davon ausgegangen, dass ein Fachmann die obige Beschreibung in weitestem Umfang nutzen kann. Die nachfolgend beschriebenen bevorzugten Ausführungsformen sind deswegen lediglich als beschreibende, keineswegs als in irgendeiner Weise limitierende Offenbarung aufzufassen.

Eine besonders bevorzugte Ausführungsform des Verfahrens ist in folgendem Schema (Tab. 1) dargestellt:

**Tab. 1 Übersicht über das erfindungsgemäße Herstellungsverfahren**

| | |
|---|---|
| 1. | Vorreinigung (optional) Hydrophobe Interaktions-Chromatographie (Phenyl-Sepharose) |
| | Laufmittel 1: 20 mM Tris/HCl, 1 M Ammoniumsulfat, pH 8.0 |
| | Laufmittel 2: dest. Wasser |
| | |
| 2. | Laufmittelwechsel für Hauptreinigung Gelfiltration (Sephadex 25) |
| | Laufmittel: 20 mM NaCl |
| | |
| 3. | Anionenaustausch-Chromatographie (Source 15Q) |
| | Laufmittel 1: 20 mM NaOH, 11 mM NaHCO₃ und 20 mM NaCl |
| | Laufmittel 2: NaCl-Gradienten (von 20mM NaOH; 11 mM NaHCO₃; 20 mM NaCl bis 20mM NaOH; 11 mM NaHCO₃; 0,5 M NaCl) |
| | |
| 4. | Hydrophobe Interaktions-Chromatographie (Source PHE) |
| | Laufmittel 1: 3 M NaCl, 20 mM NaOH, 11 mM NaHCO₃ |
| | Laufmittel 2: 20 mM NaOH |
| | |
| 5. | Gelfiltration (Superdex 75) |
| | Laufmittel: 10 mM NaOH, 11 mM NaHCO₃ und 148.4 mM NaCl |
| | |
| 6. | Neutralisierung mit 1/10 (v/v) 100 mM HCl |

Die Erfindung wird nachfolgend beispielhaft an der Reinigung des therapeutisch wirksamen rekombinanten Bet v 1 (rBet v1) dargestellt. Sämtliche Chromatographiematerialen werden von der Firma Amersham Biosciences (Freiburg, Deutschland) kommerziell vertrieben.

### Beispiel 1: Gewinnung von hypoallergenem rBet v 1

Zunächst wird ein lösliches rBet v 1 Allergen enthaltendes E. coli-Lysat nach Standardverfahren hergestellt (Breiteneder H., et al., EMBO J. 1989, 8: 1935-8; Hoffmann-Sommergruber et al., Protein Exp. Purif. 9 (1), 1997: 33-39).

Nun wird zur Entfernung von Nukleinsäuren eine Hydrophobe Interaktions-Chromatographie mit Phenyl-Sepharose in einem Tris-Ammoniumsulfat-Puffer (20 mM Tris/HCl, 1 M Ammoniumsulfat, pH 8.0) durchgeführt. Die Elution erfolgt mit destilliertem Wasser. Zur Vorbereitung auf die weiteren, mit schwach alkalischen Laufmitteln durchzuführenden Reinigungsschritte wird restliches Ammoniumsulfat gegen 20 mM NaCl mittels Gelfiltration über Sephadex G-25 ausgetauscht.

Die so vorgereinigte Proteinlösung wird zur Anionenaustausch-Chromatographie mit Source 15Q eingesetzt, wobei das Trägermaterial mit einer alkalischen Lösung (20 mM NaOH, 11 mM NaHCO₃ und 20 mM NaCl) äquilibriert ist. Der relativ hohe pH-Wert der Startlösung bewirkt, daß nahezu alle Zielproteine an den Anionenaustauscher binden. Die anschliessende Elution erfolgt mit ansteigendem NaCl-Gradienten (von 20mM NaOH; 11 mM NaHCO₃; 20 mM NaCl bis 20mM NaOH; 11 mM NaHCO₃; 0,5 M NaCl) und bewirkt eine Abtrennung von Verunreinigungen (Wirtszellproteinen) und Wirkstofffragmenten.

Der nächste Chromatographieschritt stellt eine Hydrophobe Interaktions-chromatographie mittels Source PHE dar. Dazu wird das Eluat aus der Ionenaustausch-Chromatographie auf 3 M NaCl, 20 mM NaOH, 11 mM NaHCO₃ durch Zugabe entsprechender Mengen einer 5 M NaCl-Stammlösung, einer 2 M NaOH Stammlösung und Natriumhydorgencarbonat eingestellt. Unter diesen Bedingungen bindet rBet v 1 an das Säulenmaterial. Die Elution des gebundenen Zielproteins erfolgt mit 20 mM NaOH.

Als abschließender Schritt wird eine Gelfiltration über Superdex 75, unter alkalischen Bedingungen durchgeführt. Die Chromatographielösung wird derart gewählt, daß eine Neutralisation der im Laufmittel zugesetzten Base zu der gewünschten Endformulierung führt: 10 mM NaOH, 11 mM NaHCO₃ und 148.4 mM NaCl, was den Konzentrationen einer physiologischen Kochsalzlösung entspricht.

Das Eluat der Gelfiltration wird zum Abschluss mit der zur verwendeten Base NaOH korrespondierenden Säure HCl neutralisiert, wodurch ein neutraler pH-Wert eingestellt und gleichzeitig der gewünschte Salzgehalt einer physiologischen Kochsalzlösung erzielt wird. Dies gelingt durch Zugabe von 1/10 (v/v) 100 mM HCl.

### Beispiel 2: Charakterisierung durch SDS-PAGE

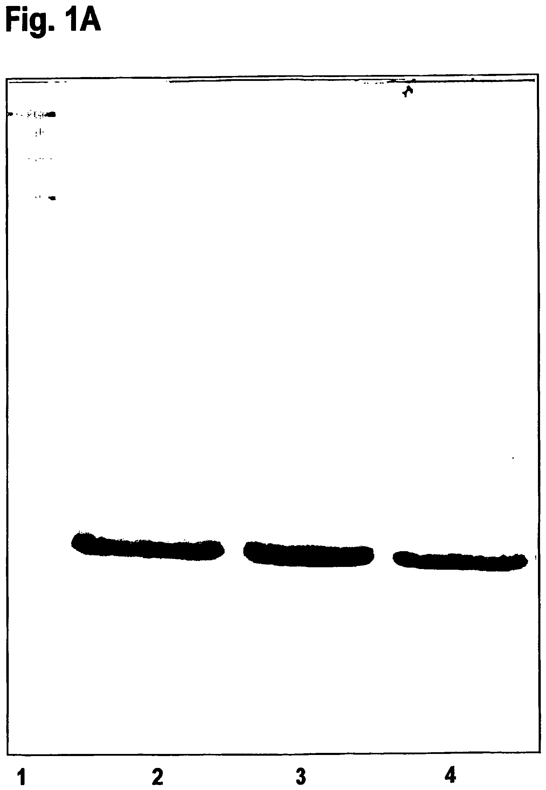

Zur Charakterisierung des hypoallergenen rBet v 1 aus Beispiel 1 wird eine SDS-PAGE (15 %) durchgeführt. Wie aus Abb. 1A ersichtlich, weisen das natürliche nBet v 1 (Spur 2), das rekombinante, herkömmlich nach Hoffmann-Sommergruber et al. (Protein Exp. Purif. 9 (1), 1997: 33-39) gereinigte rBet v 1 (Spur 4) und das erfindungsgemäß gereinigte rBet v 1 (Spur 3) in der SDS-PAGE das gleiche Molekulargewicht auf.

### Beispiel 3: Bestimmung der IgE-Aktivität mit einem Serumpool

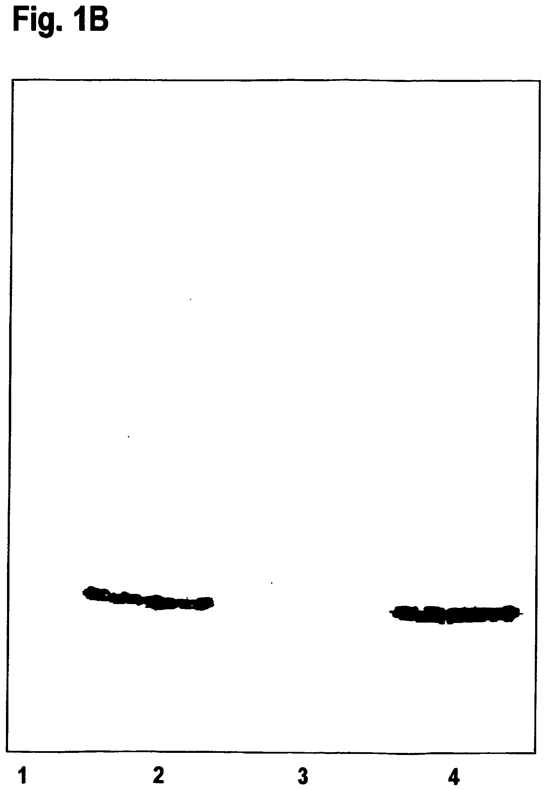

Zur Bestimmung der IgE-Aktivität wird die SDS-PAGE aus Beispiel 2 auf Nitrozellulose geblottet. Nach Versetzen des Blots mit einem Blutserumpool von Birkenpollenallergikern wird mit einem Konjugat bestehend aus einem Anti-IgE-Antikörper und Alkalischer Phosphatase inkubiert. Die durch die Alkalische Phosphatase vermittelte Farbreaktion (Abb. 1 B) zeigt eine IgE-Aktivität des natürlichen nBet v 1 sowie des rekombinanten, herkömmlich gereinigten rBet v 1, nicht aber des erfindungsgemäß gereinigten rBet v 1-MF.

### Beispiel 4: Bestimmung der IgE-Aktivität mit individuellen Seren

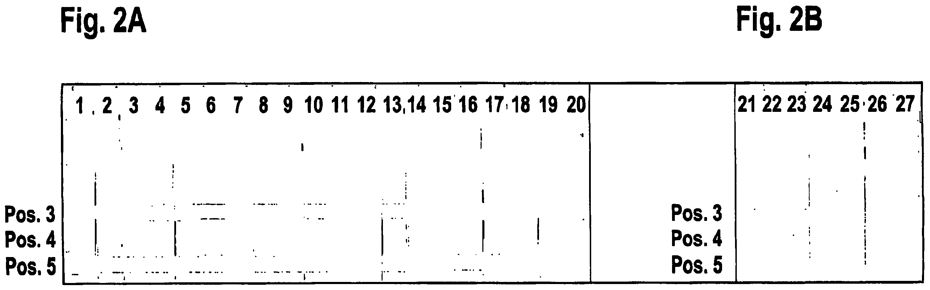

Zur Bestimmung der IgE-Aktivität mit Blutseren von individuellen Birkenpollenallergikern werden gemäß Abbildung 2A nBet v 1 (Position 3), das rekombinante, herkömmlich gereinigte rBet v 1 (Position 5) sowie das erfindungsgemäß gereinigte rBet v 1 (Position 4) auf eine Nitrozellulosemembran aufgebracht und analog Beispiel 3 untersucht. Abb. 2A zeigt, daß mit der Ausnahme von Serum 5, wo das erfindungsgemäß gereinigte rBet v 1 eine schwache IgE-Aktivftät aufweist, nur das natürliche nBet v 1 und das rekombinante, herkömmlich gereinigte rBet v 1, nicht aber das erfindungsgemäß gereinigte rBet v 1 IgE-aktiv sind.

Zum Nachweis der Identität der untersuchten Allergene wurde die Nitrozellulosemembran mit verschiedenen polyklonalen Kaninchen-Anti-Bet v 1-Antikörpern (Proben 21 bis 26) sowie mit dem monoklonalen Maus-Anti-Bet v 1-Antikörper 6B6 (Probe 27) inkubiert und anschließend analog Beispiel 3 behandelt (Abb. 2B).

### Beispiel 5: Quantifizierung der IgE-Bindung

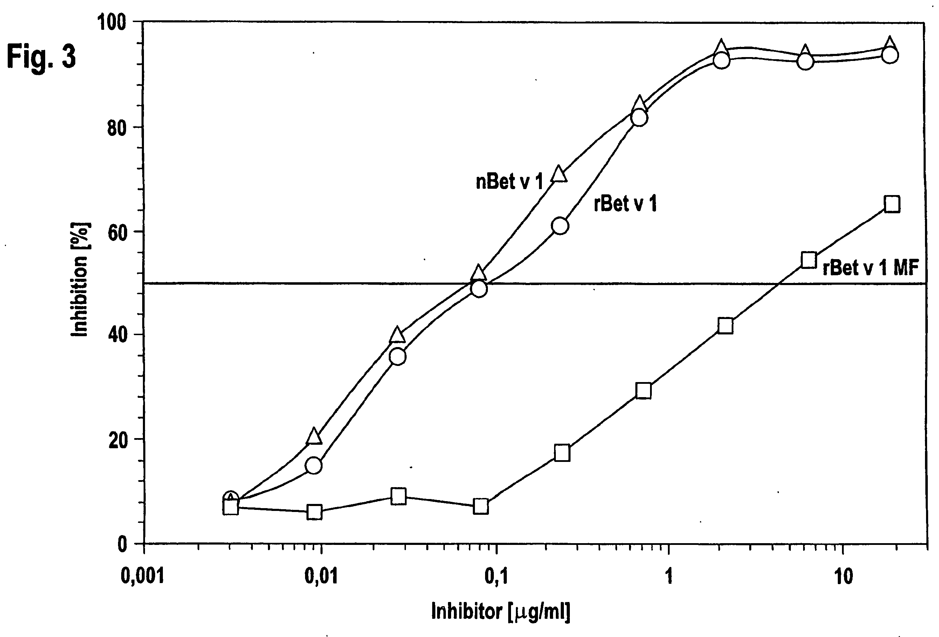

In einem gemäß Suck et al. (Int. Arch. Allergy Immunol. 2000; 121: 284-291) durchgeführten EAST-Inhibitionsassay mit einem Allergiker-Serumpool werden das natürliche nBet v 1, das rekombinante, herkömmlich gereinigte rBet v 1 und das erfindungsgemäß gereinigte rBet v 1 hinsichtlich der Stärke ihrer IgE-Bindung miteinander verglichen (Abb. 3). Es zeigt sich, daß das erfindungsgemäß gereinigte rBet v 1 in seiner IgE-Aktivität gegenüber den anderen Bet v 1 Proteinen mehr als 100fach reduziert ist

### Beispiel 6: Bestimmung der T-Zell-Stimulierung

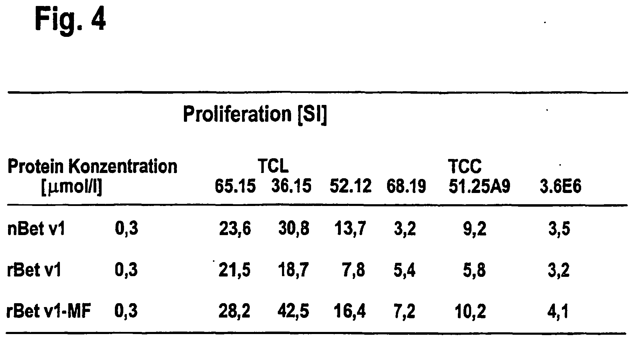

Zur Bestimmung des Einflusses des erfindungsgemäßen rBet v 1 Allergens auf das Wachstum von T-Zellen wird ein Proliferationsassay mit T-Zell-Linien (TCL) und T-Zell-Klonen (TCC) gemäß Schramm et al. (1999, J. Immunol. 162 (4): 2406-2414) durchgeführt (Abb. 4). Aus dem Vergleich der Stimulationsindices (SI) ergibt sich, daß die T-Zellen der untersuchten Spender mit rBet v 1 mindestens ebenso stark reagieren wie mit dem natürlichen nBet v 1 oder dem herkömmliche gereinigten rekombinanten rBet v 1. In Abhängigkeit von den gewählten Bedingungen übersteigt die Reaktion mit rBet v 1 die Reaktion mit nBet v 1 bzw. rBet v 1 sogar um bis zu ein Drittel.

## Patentansprüche

1. Verfahren zur Herstellung von hypoallergenem Birkenpollenhauptallergen rBet v 1 mittels eines oder mehrerer chromatographischer Reinigungsschritte unter Verwendung von im wesentlichen ungepufferten wässrigen Basen als Laufmittel und anschließender Neutralisation, **dadurch gekennzeichnet, dass** als Ausgangsstoff ein rekombinant hergestelltes lösliches rBet v 1 Rohprotein eingesetzt wird.

2. Verfahren nach Anspruch 1, **dadurch gekennzeichnet, dass** das rekombinant hergestellte lösliche rBet v 1 Rohprotein in E. coli exprimiert wurde.

3. Verfahren nach Anspruch 1 oder 2, **dadurch gekennzeichnet, dass** die chromatographischen Reinigungsschritte Anionenaustausch-Chromatographie, Hydrophobe Interaktions-Chromatographie und Gelfiltration umfassen.

4. Verfahren nach einem oder mehreren der Ansprüche 1 bis 3, **dadurch gekennzeichnet, daß** die chromatographischen Reinigungsschritte in folgender Reihenfolge ausgeführt werden: erste Gelfiltration, Anionenaustausch-Chromatographie, Hydrophobe Interaktions-Chromatographie, zweite Gelfiltration.

5. Verfahren nach einem oder mehreren der Ansprüche 1 bis 4, **dadurch gekennzeichnet, daß** die als Laufmittel verwendete Base in einem Konzentrationsbereich von 5 mM bis 100 mM, vorzugsweise 5 mM bis 40 mM eingesetzt wird.

6. Verfahren nach einem oder mehreren der Ansprüche 1 bis 5, **dadurch gekennzeichnet, daß** die als Laufmittel verwendete Base NaOH ist.

7. Verfahren nach einem oder mehreren der Ansprüche 1 bis 6, **dadurch gekennzeichnet, daß** dem Laufmittel ein anorganisches Neutralsalz und gegebenenfalls NaHCO₃ zugesetzt werden.

8. Verfahren nach einem oder mehreren der Ansprüche 1 bis 7, **dadurch gekennzeichnet, daß** das dem Laufmittel zugesetzte anorganische Neutralsalz NaCl ist.

9. Verfahren nach einem oder mehreren der Ansprüche 6 bis 8, **dadurch gekennzeichnet, daß** die Konzentrationen der in dem Laufmittel enthaltenen Substanzen NaOH, NaCl und NaHCO₃ so gewählt werden, daß in dem die Reinigung abschließenden Neutralisationsschritt die für eine physiologische Kochsalzlösung typischen Konzentrationen eingestellt werden können.

10. Verfahren nach einem oder mehreren der Ansprüche 1 bis 9, **dadurch gekennzeichnet, daß** das rBet v 1 Rohprotein in einer gepufferten Lösung chromatogaphisch und/oder durch eine Salzfällung vorgereinigt wird.

11. Verfahren zur Reduzierung der IgE-Aktivität des Birkenpollenhauptallergens rBet v 1, **dadurch gekennzeichnet, daß** ein rekombinant hergestelltes lösliches Birkenpollenhauptallergen rBet v 1 einem der in den Ansprüchen 1 bis 10 beschriebenen Verfahren unterzogen wird.

## Claims

1. Process for the preparation of hypoallergenic major birch pollen allergen rBet v 1 by means of one or more chromatographic purification steps using essentially unbuffered aqueous bases as eluent and subsequent neutralisation, **characterised in that** the starting material employed is a soluble rBet v 1 crude protein prepared by recombinant methods.

2. Process according to Claim 1, **characterised in that** the soluble rBet v 1 crude protein prepared by recombinant methods was expressed in E. coli.

3. Process according to Claim 1 or 2, **characterised in that** the chromatographic purification steps include anion exchange chromatography, hydrophobic interaction chromatography and gel filtration.

4. Process according to one or more of Claims 1 to 3, **characterised in that** the chromatographic purification steps are carried out in the following sequence: first gel filtration, anion exchange chromatography, hydrophobic interaction chromatography, second gel filtration.

5. Process according to one or more of Claims 1 to 4, **characterised in that** the base used as eluent is employed in a concentration range from 5 mM to 100 mM, preferably 5 mM to 40 mM.

6. Process according to one or more of Claims 1 to 5, **characterised in that** the base used as eluent is NaOH.

7. Process according to one or more of Claims 1 to 6, **characterised in that** an inorganic neutral salt and optionally NaHCO₃ are added to the eluent.

8. Process according to one or more of Claims 1 to 7, **characterised in that** the inorganic neutral salt added to the eluent is NaCl.

9. Process according to one or more of Claims 6 to 8, **characterised in that** the concentrations of the substances NaOH, NaCl and NaHCO₃ present in the eluent are selected in such a way that the typical concentrations for physiological saline solution can be set in the neutralisation step completing the purification.

10. Process according to one or more of Claims 1 to 9, **characterised in that** the rBet v 1 crude protein is pre-purified in a buffered solution by chromatography and/or by salt precipitation.

11. Process for reducing the IgE activity of major birch pollen allergen rBet v 1, **characterised in that** a soluble major birch pollen allergen rBet v 1 prepared by recombinant methods is subjected to one of the processes described in Claims 1 to 10.

## Revendications

1. Procédé pour la préparation d'allergène de pollen de bouleau principal hypoallergénique rBet v 1 au moyen d'une ou plusieurs étapes de purification chromatographique utilisant des bases aqueuses essentiellement non tamponnées en tant qu'éluent et de neutralisation qui s'ensuit, **caractérisé en ce que** le matériau de départ employé est une protéine fraîche rBet v 1 soluble préparée par des procédés recombinants.

2. Procédé selon la revendication 1, **caractérisé en ce que** la protéine fraîche rBet v 1 soluble préparée par des procédés recombinants a été exprimée dans E. coli.

3. Procédé selon la revendication 1 ou 2, **caractérisé en ce que** les étapes de purification chromatographique incluent une chromatographie par échange d'anions, une chromatographie par interaction hydrophobe et une filtration sur gel.

4. Procédé selon une ou plusieurs des revendications 1 à 3, **caractérisé en ce que** les étapes de purification chromatographique sont mises en oeuvre dans la séquence suivante : première filtration sur gel, chromatographie par échange d'anions, chromatographie par interaction hydrophobe, seconde filtration sur gel.

5. Procédé selon une ou plusieurs des revendications 1 à 4, **caractérisé en ce que** la base utilisée comme éluent est employée dans une plage de concentrations de 5 mM à 100 mM, de préférence 5 mM à 40 mM.

6. Procédé selon une ou plusieurs des revendications 1 à 5, **caractérisé en ce que** la base utilisée comme éluent est NaOH.

7. Procédé selon une ou plusieurs des revendications 1 à 6, **caractérisé en ce qu'**un sel neutre inorganique et optionnellement NaHCO₃ sont ajoutés à l'éluent.

8. Procédé selon une ou plusieurs des revendications 1 à 7, **caractérisé en ce que** le sel neutre inorganique ajouté à l'éluent est NaCl.

9. Procédé selon une ou plusieurs des revendications 6 à 8, **caractérisé en ce que** les concentrations des substances NaOH, NaCl et NaHCO₃ présentes dans l'éluent sont choisies de telle sorte que les concentrations typiques pour la solution saline physiologique peuvent être établies dans l'étape de neutralisation parachevant la purification.

10. Procédé selon une ou plusieurs des revendications 1 à 9, **caractérisé en ce que** la protéine fraîche rBet v 1 est pré-purifiée dans une solution tamponnée par chromatographie et/ou par précipitation de sel.

11. Procédé pour réduire l'activité IgE d'allergène de pollen de bouleau principal rBet v 1, **caractérisé en ce qu'**un allergène de pollen de bouleau principal rBet v 1 soluble préparé par des procédés recombinants est soumis à l'un des procédés décrits dans les revendications 1 à 10.
